Europäisches Patentamt

European Patent Office

Office européen des brevets

(19)

(11) Veröffentlichungsnummer: **0 194 224**
**B1**

(12) **EUROPÄISCHE PATENTSCHRIFT**

(45) Veröffentlichungstag der Patentschrift: **27.12.90**

(21) Anmeldenummer: **86730031.1**

(22) Anmeldetag: **24.02.86**

(51) Int. Cl.⁵: **A 61 N 1/365,** A 61 B 5/02,
H 04 R 1/46

(54) **Herzschrittmacher mit physiologischer Steuerung.**

(30) Priorität: **22.02.85 DE 3506791**

(43) Veröffentlichungstag der Anmeldung:
**10.09.86 Patentblatt 86/37**

(45) Bekanntmachung des Hinweises auf die
Patenterteilung:
**27.12.90 Patentblatt 90/52**

(84) Benannte Vertragsstaaten:
**AT CH DE FR GB LI NL SE**

(56) Entgegenhaltungen:
EP-A-0 080 348
EP-A-0 118 306
WO-A-82/03782
DE-A-2 703 781
DE-A-3 240 430
US-A-4 140 132

(73) Patentinhaber: **BIOTRONIK Mess- und
Therapiegeräte GmbH & Co Ingenieurbüro
Berlin
Sieversufer 8
D-1000 Berlin 47 (DE)**

(72) Erfinder: **Schaldach, Max, Prof. Dr.-Ing.
Turnstrasse 5
D-8520 Erlangen (DE)**

(74) Vertreter: **Christiansen, Henning, Dipl.-Ing.
Patentanwalt CHRISTIANSEN Pacelliallee 43/45
D-1000 Berlin 33 (DE)**

Courier Press, Leamington Spa, England.

## Beschreibung

Die Erfindung betrifft einen Herzschrittmacher der im Oberbegriff des Anspruchs 1 angegebenen Art.

Es sind verschiedentlich Versuche unternommen worden, künstliche Herzschrittmacher bezüglich der Stimulationsfrequenz an die augenblicklichen physiologischen Bedürfnisse des Patienten anzupaßbar auszubilden.

Physiologische Steuerung bedeutet dabei eine Beeinflussung des Schrittmachers zur Anpassung der Leistungsfähigkeit des Herzens entsprechend dem augenblicklichen Bedarf, d.h. insbesondere bezüglich seiner Grundrate, die unabhängig ist von der bisherigen patientenabhängigen Steuerung durch im Herzen abgeleitete elektrische Signale, welche allein eine Vermeidung der Konkurrenz von stimulierten Impulsen mit Spontanaktionen zum Ziele hat.

Diese bisherigen Versuche, eine derartige Steuerung der Stimulation in breitem Maße anzuwenden scheitern daran, daß meist nur ein unscharfer Zusammenhang zwischen der verwendeten Meßgröße und der die Herzleistung bestimmenden Stimulationsrate besteht oder Schwierigkeiten vorhanden sind, geeignete Meßwertaufnehmer zu finden, die für einen wartungsfreien Betrieb über längere Zeiträume — und insbesondere zur Implantation in den menschlichen Körper — in Betracht kommen. Zu den bisher zur bedarfsabhängigen Steuerung herangezogenen physiologischen Meßgrößen gehören die Blutsauerstoffsättigung die Bluttemperatur und das QT-Intervall (vgl. hier beispielsweise EP 0 007 189 B1).

Bei den bisher verwendeten Meßgrößen ist eine direkte, patientenindividuelle Einstellung des Zusammenhangs zwischen Meßgröße und stimulierter Herzfrequenz nicht möglich, da beim kardiovaskulären Regelsystem des Menschen zwischen den eine körperliche Belastung anzeigenden Parametern und der daraus resultierenden Herzleistung komplexe funktionale zeitabhängige Abhängigkeiten zu berücksichtigen sind. Für eine exakte Steuerung entsprechend jedem einzelnen Belastungsfall des Patienten wäre für den patientenindividuellen Abgleich jedes Mal das Erreichen eines stationären Zustandes erforderlich, um eine definierte Zuordnung von Steuerparameter und Herzrate zu ermöglichen. Da die zeitliche Belastbarkeit von Patienten aber u.a. wegen der sich erschöpfenden körperlichen Reserven — insbesondere bei vorhandener Herzkrankheit — zeitlich begrenzt ist, kann der notwendige stationäre Zustand gar nicht im notwendigen Umfang erreicht werden. Wegen der im übrigen komplexen physiologischen Zusammenhänge ist die Steuerung der Schrittmacherfrequenz gemäß den bekannten Anordnungen daher mit erheblichen Abweichungen im Nachführungsbereich der Frequenz behaftet, so daß die physiologische Anpassung der Stimulationsrate nicht mit der wünschenswerten Zuverlässigkeit gewährleistet ist.

Der Erfindung liegt die Aufgabe zugrunde, einen Steueralgorithmus aus einer physiologischen Größe herzuleiten, welche einen Zusammenhang zur Herzfrequenz aufweist und somit eine genauere Nachführung der Herzfrequenz entsprechend den Bedürfnissen des Patienten ermöglicht.

Die Erfindung beruht dabei insbesondere auf der Erkenntnis, daß die systolischen Zeitintervalle (vgl. den Aufsatz in Basic Res. Cardiol. 73, 85—96 (1978) con G. K. Wolf, G. G. Belz und M. Stauch: "Systolic time intervals — Correction for heart rate") einen funktionalen Zusammenhang mit der Herzfrequenz, d.h. mit dem RR-Abstand aufweisen, so daß eine direkte Verarbeitung von Zeitbeziehungen möglich ist.

Im Gegensatz zu den bisherigen Untersuchungen, welche die Herzrate dazu benutzen, um die bei einem Patienten ermittelten systolischen Zeitintervalle zu korrigieren, werden hier diese Zeitintervalle dazu benutzt, um die Stimulationsrate eines küntslichen Herzschrittmachers zu variieren. Als systolische Intervalle (STI — Systolic Time Intervals) kommen dabei bevorzugt die Größen PEP (Pre-Ejection Period), LVET (Left Ventricular Ejection Time), der Quotient der vorgenannten Größen und diese Größen gegebenenfalls in Kombination mit den im Herzen abzuleitenden für die elektrische Systole kennzeichnenden Impulsen in Betracht.

Die Lösung beruht dabei insbesondere auf folgenden Zusammenhängen:

Die systolischen Zeitintervalle weisen einen im wesentlichen linearen Zusammenhang mit dem RR-Abstand auf. Dieser Zusammenhang kann für den betreffenden Patienten mit einem einmaligen Durchlaufen des in Betracht kommenden Herzfrequenzbereiches durch ergonometrische Belastung aufgenommen werden, wobei die Stimulationsrate jeweils auf einen der Belastung in etwa entsprechenden Wert eingestellt bzw. ein aus einem derartigen Bereich physiologisch günstig erscheinender Wert ausgewählt und dem entsprechenden physiologischen Zeitintervall in einem digitalen Datenspeicher zugeordnet wird. Diese Werte bilden Stützstellen bei einer im Betrieb erfolgenden Interpolation.

Entsprechend kann bei einer bevorzugten Ausführung der Erfindung auch bei vorhandenen Spontanaktionen des Ventrikels — gegebenenfalls separat — eine Zuordnung der jeweiligen systolischen Zeitintervalle zu verschiedenen Spontanfrequenzen des Herzens festgehalten werden. Die Frequenzwerte lassen sich ebenfalls — gegebenenfalls unter Berücksichtigung einer die Abweichung der im Herzen vorliegenden physiologischen Verhältnisse zwischen stimulierten und spontanen Aktionen kompensierenden Größe als Stützstellen bzw. Kontrollgrößen verwenden. Eine Aussage über eine eventuelle Abweichung der systolischen Intervalle bei Schrittmacherintervention im Vergleich zu Spontanaktionen läßt sich insbesondere beim Übergang von spontanen zu stimulierten Herzaktionen unter Bezugnahme auf die jeweils letzte Spontanfrequenz erzielen. Dazu wird vorzugsweise nach Beendigung des Belastungszyklus bei abfallender Herzfrequenz des Patienten der Schrittmacher jeweils zeitweise im Demand-Betrieb der abfallenden Herzfrequenz derart eng nachgeführt, daß mit der absinkenden spontanen Herzaktivität in regelmäßigen Abständen im Frequenzraster Stimulationen erzwungen werden, welche das Herz des Patienten jeweils kurzfristig mit der kurz vorher verlassenen höheren (ehemaligen Spontan-)Frequenz stimulieren. Auf diese Weise läßt sich für den gesamten in Betracht kommenden Frequenzbereich der funktionale Zusammenhang mit den systolischen Intervallen bei stimuliertem Betrieb festhalten, so daß für diesen Betriebszustand bei Ausfall von Spontanaktionen für jeden Betriebsfall erkannt werden kann, ob die augenblickliche Stimulationsfrequenz den augenblicklichen physiologischen Bedingungen entspricht.

Durch entsprechende Speicherung der systolischen Intervalldaten in der jeweiligen Stimulationsfrequenz zugeordneten Speicherplätzen kann somit später erkannt werden, welche Stimulationsfrequenz aufgrund der augenblicklichen physiologischen Bedingungen angestrebt werden sollte.

Die Heranziehung der systolischen Zeitintervalle als physiologische Steuergröße für die Herzfrequenz enthält noch weitere vorteilhafte Möglichkeiten: Die systolischen Zeitintervalle beinhalten nämlich nicht nur eine Information bezüglich der augenblicklich physiologisch günstigen Herzrate, sondern sind außerdem noch von der augenblicklichen Kammerfüllung — und damit von erzielbaren Schlag — und somit Minutenvolumen — abhängig. Das sich für eine augenblickliche Stimulationsfrequenz einstellende Zeitintervall bildet also nicht direkt ein Maß für den augenblicklichen Frequenzbedarf, da bei Stimulation mit einer dieser "physiologisch korrekten" Frequenz abweichenden Frequenz die Kammerfüllung bei der aktuellen Frequenz das systolische Zeitintervall mit beeinflußt und somit nur mittelbar auf die einzustellende Zielfrequenz geschlossen werden kann.

Bei einer stufenweisen Annäherung erfolgt eine Approximation an die Zielfrequenz in kleinen Schritten, wobei sich durch den fortgesetzten Vergleich der Abweichungsrichtung der systolischen Zeitintervalle mit der Änderungsrichtung eine zusätzliche Kontrolle über die Korrektheit der vorgesehen Änderung ergibt. Eine weitere Kontrollmöglichkeit ist durch Vergleich der erreichten Zielfrequenz mit den für diese Zielfrequenz festgehaltenen Absolutwerten der systolischen Zeitintervalle gegeben.

Diese Überlegungen gelten für den Fall, daß keine Spontanaktionen auftreten, welche einen direkten Rückschluß auf die vom Herzen selbst angestrebte Rate zulassen. Um eine derartige Anpassung zu ermöglichen, ist es günstig, die Tendenz der auf diese Weise erzielten, den physiologischen Verhältnissen angepaßte Stimulation geringfügig niedriger zu halten, als die den entsprechenden Verhältnissen zugehörige natürliche Frequenz, da anderenfalls möglichen Spontanaktionen vielfach durch unnötige Stimulationen vorgegriffen würde.

Bei der Ansnutzung aller Möglichkeiten des hier beschriebenen Verfahrens lassen sich insbesondere beim ischämischen Herzen, bei dem der Zusammenhang zwischen Herzminutenvolumen und -frequenz nichtlinear ist und aufgrund der vorhandenen Schädigung Schwankungen unterliegt Vorteile erzielen, wobei bestimmte eine gute Kammerfüllung gewährleistende Herzfrequenzen zu bevorzugen sind.

Während bei Patienten mit intaktem Myokard der Zusammenhang zwischen systolischen Zeitintervallen und Herzfrequenz angenähert linear ist, ergibt sich im Falle der bei geschädigtem Myokard nur eine herabgesetzte Kammerfüllung ermöglichenden Frequenzen eine Verschiebung zu kleineren Intervallen, so daß sich ein "ausgebeulter" Verlauf der resultierenden Abhängigkeit ergibt. Bei Auswahl einer Stimulationsfrequenz aufgrund der ermittelten systolischen Zeitintervalle sind somit solche Frequenzbereiche zu bevorzugen, welche bezogen auf eine einen im wesentlichen linearen Verlauf angebenden gedachten Linie möglichst große absolute LVET-Werte aufweisen. Bei Veränderung der stimulierten Frequenz aufgrund sich ändernder Intervalle wäre unter Berücksichtigung der zuvor beschriebenen schrittweisen Annäherung erst eine solche Intervalländerung abzuwarten, die zu einem neuen Frequenzwert führt, der ebenfalls eine günstige Kammerfüllung erwarten läßt. Derartige "günstige" Stimulationsraten könnten aber auch in anderer Weise bevorzugt ausgewählt werden. Dabei ist es unbenommen, die tatsächlich ausgeführte Stimulationsfrequenzänderung in kleinen Schritten vorzunehmen und dabei die oben erwähnten Plausibilitätskontrollen durchzuführen, welche die gewählte Änderungsrichtung und -weite bestätigen. Praktisch ausgeführt wird eine derartige Steuerung dadurch, daß in dem zugeordneten Datenspeicher diejenigen Werte für die Stimulations-Grundrate ersetzt werden durch Daten, die den in diesen Fällen auszuwählenden Frequenzen zugeordnet sind.

Da die systolischen Intervalle zusätzlichen Schwankungen unterliegen, sind die hier beschriebenen Verfahren bevorzugt in der weise als statistisch anzuwenden, daß für eine Änderung der Stimulationsfrequenz bewirkenden Entscheidungen stets eine Folge von systolischen Zeitintervallen bzw. ein in Zeitabständen aufgenommenes Muster gewählt wird, bei dem die erhaltenen Werte gemittelt werden, um die Zuverlässigkeit des Verfahrens zu erhöhen. Dabei können zusätzlich vorteilhafte Maßnahmen statistischer oder digitaler Regelverfahren angewendet werden, wie z.B. die zeitliche Veränderung der Schrittweite nach Art der Delta-Modulation.

Die Zuverlässigkeit des hier dargestellten Verfahrens läßt sich ohne Schwierigkeit durch die erreichte Konvergenz der Änderungsschritte abschätzen. Die Sicherheit des Patienten ist in jedem Fall dadurch gewährleistet, daß durch Kontrollmittel, welche die Änderungsrichtungen und die Zahl der Änderungen bzw. deren Plausibilität kontrollieren, sine Umschaltung zu einem Betrieb mit fester Grundrate veranlassen.

Das hier beschriebene Verfahren läßt sich auf in Verschiedenen Modes abreitende Schrittmacher anwenden, wobei der Einkammerschrittmacher zunächst bevorzugt wird. Bei einem Wechsel von der ventrikulären zu AV-sequentiellen Stimulation ist zu berücksichtigen, daß in beiden Fällen wegen des unterschiedlichen Grads der Kammerfüllung bzw. der vom Stimulationmode abhängigen systolischen Intervalle getrennte funktionale Abhängigkeiten zu berücksichtigen sind, die in seperaten Speichern festgehalten werden müssen. Da im übrigen die Verhältnisse analog sind, kann — jeweils bezogen auf einander entsprechende Stimulationsfrequenzen — beliebig von einem Mode zum anderen gewechselt werden, wenn die Verhältnisse es erfordern.

Die automatische Anpassung der Stimulationsfrequenz läßt sich durch Programmierung bezüglich der in Betracht kommenden Frequenzgrenzen beeinflussen. Bei der automatischen Adaption entsprechend einem "Selbstlernvorgang", der auch ohne Durchlaufen eines Belastungszyklus unter Beaufsichtigung eines Arztes aufgrund der sich bei der normalen Tätigkeit Belastungsänderungen möglich ist, würde der Steuerbereich für stimulierte Frequenzen demjenigen angepaßt, der jeweils unter Auftretten von Spontanaktionen durchlaufen wurde.

Die Funktion des auf die dargestellte Weise frequenzgesteuerten Schrittmachers wäre für den behandelnden Arzt bei Ausnutzung der Möglichkeiten der Zweiweg-Kommunikation jederzeit ohne weiteres einsichtig. Günstigerweise ist dazu bei der externen Kommunikationseinheit ein LCD-Display vorzusehen, auf dem sich die funktionale Abhängigkeit von den systolischen Zeitintervallen und Stimulationsfrequenz (RR-Abstand), in den innerhalb des Schrittmachers gespiecherten Werte graphisch darbietet. Die Grad der sich hier ergebenden Linearität der erfaßten Abhängigkeiten bei einem oder mehreren Belastungszyklen in der der oben angegebenen Weise, geben dem Arzt in anschaulicher Weise Aufschluß über die Möglichkeiten des hier beschriebenen Verfahrens im Hinblick auf den jeweiligen Patienten. Durch Berücksichtigung von Krümmungen des sich darbietenden funktionalen Zusammenhangs lassen sich extern in entsprechenden dazu reservierten Speicherplätzen Vorzugsfrequenzen manuell programmieren, welche dem Arzt für den bestimmten Anwendungsfall günstig erscheinen. Durch die individuelle Programmierbarkeit der funktionalen Abhängigkeit von Stimulationsfrequenz als Grundrate bei der Demandfunktion oder auch bei einem Schrittmacher ohne diese Eigenschaft kann verschiedensten Krankheitsbildern Rechnung getragen werden.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 ein Blockschaltbild eines Ausführungsbeispiels des erfindungsgemäßen Herzschrittmachers,

Figur 1a ein Detail des Blockschaltbilds gemäß Figur 1,

Figur 2 ein Kommunikationsteil für die externe Abfrage und Steuerung des Schrittmachers durch den Arzt,

Figur 3 einen akustischen Meßwertaufnehmer in vergrößerter Wiedergabe im Schnitt.

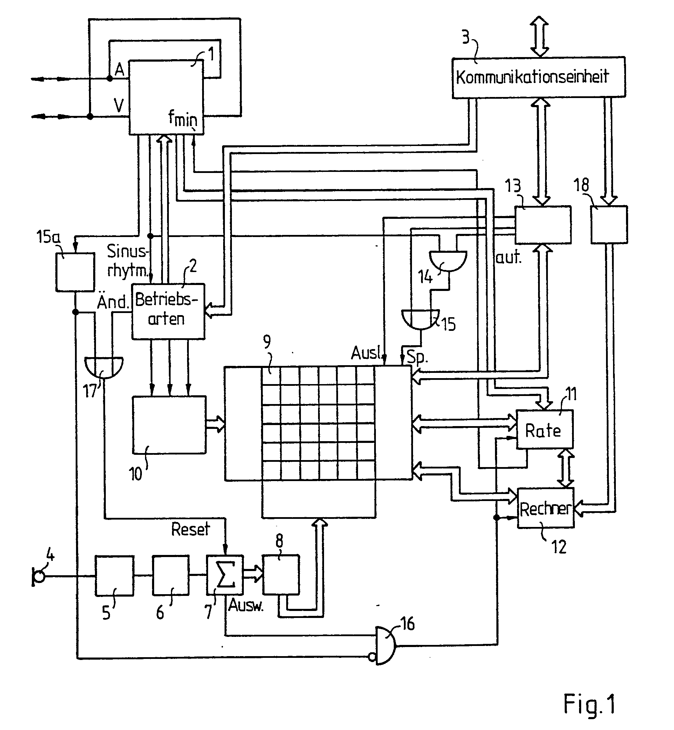

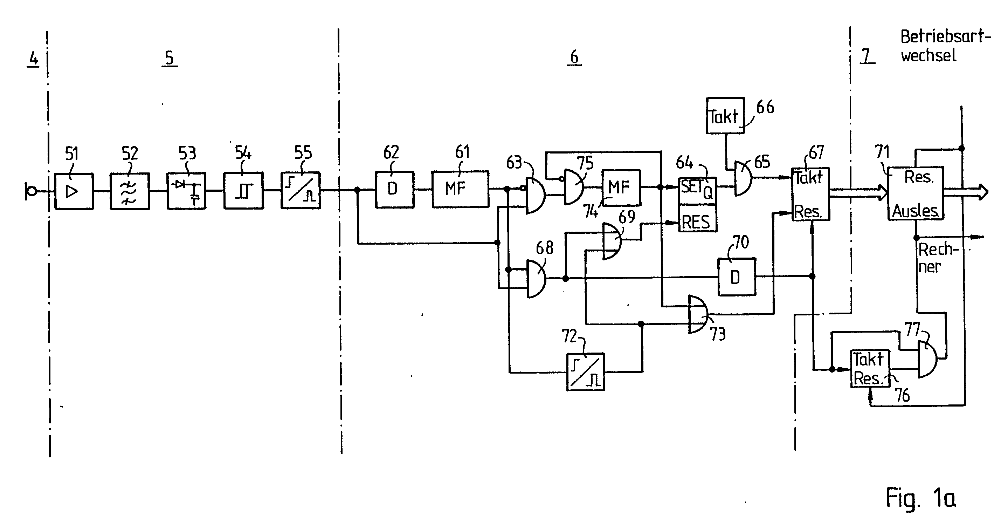

Das in Figur 1 wiedergegebene Blockschaltbild des Ausführungsbeispiels enthält einen konventionellen programmierbaren Atrium- und/oder Ventrikel-Schrittmacher mit Demand-Funktion. Die entsprechenden Ein- und Ausgänge zur Verbindung mit der Atrium- bzw. Ventrikel-Elektrode sind mit "A" und "V" gekennzeichnet. Der Betriebszustand des Steuerbausteins 1 für die Grundfunktionen des Schrittmachers wird durch eine Schalteinheit 2 für die Betriebszustände (Modes) des Schrittmachers bestimmt, welche über externe Programmerimittel mittels einer Kommunikationseinheit 3 beeinflußbar ist. Durch die Schalteinheit 2 lassen sich also verschiedene Betriebszustände (VVI-, DAT-, DDD-Mode, etc.) einstellen. Die bei den bisherigen multiprogrammierbaren Schrittmachern von außen einstellbare Grundrate oder Interventionsfrequenz läßt sich bei dem hier dargestellten Schrittmacher durch externe Programmierung in den Betriebszustand "Physiologische Steuerung" durch im Körper aufgenommene Signale selbsttätig den Bedürfnissen des Patienten anpassen.

Als Besonderheit enthält der hier dargestellte Steuerbaustein somit einen Anschluß $f_{min}$ zur Beeinflussung der Grundfrequenz. Die übrigen in Figur 1 wiedergegebenen Baugruppen sind ebenfalls in dem implantierbaren Gehäuse des Schrittmachers zusammengefaßt.

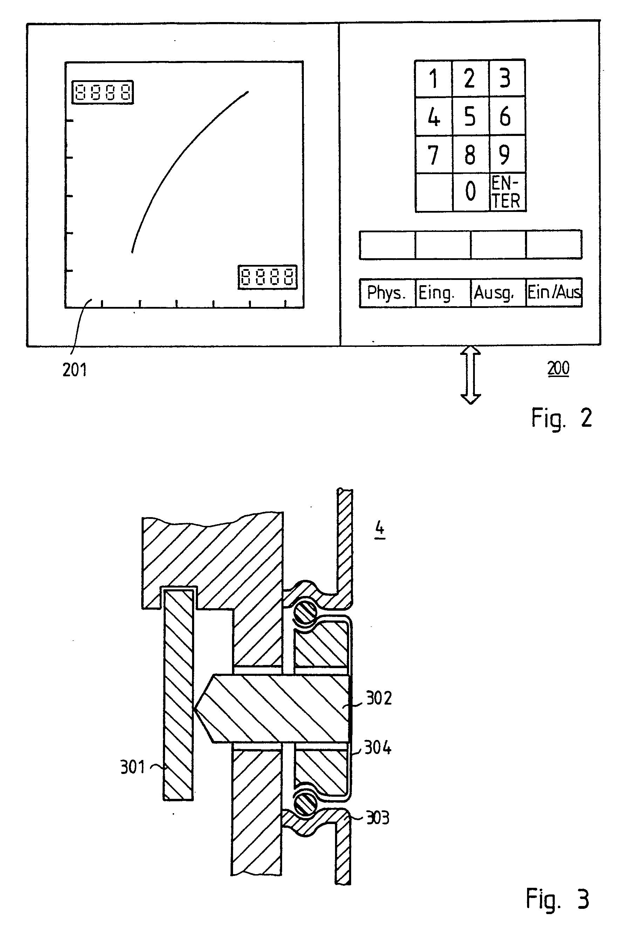

Die Grundrate, d.h. das Escape-Intervall, beginnend mit einer vorangegangenen stimulierten oder spontanen Atrium- bzw. Ventrikelaktion und endend mit dem Zeitpunkt, in dem bei ausfallender Spontanaktion im Atrium- bzw. Ventrikel stimuliert wird, ist durch die übrige dargestellte Anordnung veränderbar. Die Steuerung erfolgt in Abhängigkeit von innerhalb des Körpers des Patienten akustisch aufgenommenen Signalen, den linksventrikulären systolischen Intervallen. Diese Signale werden mittels eines Mikrofons 4 aufgenommen, welches als Körperschallempfänger ausgebildet ist und bevorzugt einen piezokeramischen Schallwandler enthält. Die Einzelheiten eines zur Ausführung der Erfindung geeigneten Schallaufnehmers werden anhand von Fig. 3 näher dargestellt. Das Ausgangssignal des Schallaufnehmers wird einem Verstärker 5 zugleitet, welcher eine Ausfilterung der relevanten Frequenzbereiche vornimmt und einen Ausgangssignal abgibt, wenn Signale der ausgewählten Frequenzen für einen vorgegebenen Mindestzeitraum anstehen. In Betracht kommen vorzugsweise Frequenzen, wie sie bisher zur phonokardiologischen Untersuchung der systolischen Intervalle hernagezogen wurden.

Das Ausgangssignal der Stufe 5 wird einem Intervalldiskriminator 6 zugeleitet, welcher — gestartet durch die Vorderflanke eines ersten Eingangsimpulses — die Zeitdauer bis zu einem nachfolgenden Impuls ermittelt, wobei jeweils durch den ersten eingehenden Impuls ein Zurücksetzen erfolgt und die Auswertung auf ein vorgegebenes Zeitfenster begrenzt ist, nach deren erfolglosem Ablauf erneut ein Zurücksetzen der Meßeinrichtung vorgenommen wird. Erscheinen jedoch zwei aufeinanderfolgende Impulse innerhalb des Zeitfensters, so wird der zeitliche Abstand der Vorderflanken als digitales Signal ausgegeben und einem Mittelwertspeicher 7 zugeführt, welcher aus einer Anzahl von aufeinanderfolgenden Zeitintervallwerten einen Mittelwert bildet und diesen Mittelwert festhält.

Der Mittelwert wird bei einem Wechsel der eingestellten Betriebsart des Schrittmachers (auch durch externe Programmierung) oder aber durch einen Wechsel des Stimulationszustandes (Übergang vom Betrieb mit Herzeigenaktion zum stimulierten Betrieb zurück, etc.) zurückgesetzt, so daß eine Mittelwertbildung der aufeinanderfolgenden systolischen Intervalle nur für gleichchartige Betriebszustände erfolgt.

Das Ausgangssignal des Mittelwertspeichers 7 wird in einem Adressdecoder 8 übertragen, wobei die vom Decoder angewerteten Adressen durch die gewählte Digitalisierung entsprechend der kleinsten Stufung der Digitalwerte gequantelt ist, so daß die Auflösung der aufzuwertenden Zeitintervalle jeweils die Differenz zweier benachbarter Speicheradressen bildet. Der Adressdecoder bewirkt noch zusätzlich insofern eine Umformung der Ausgangssignal des Mittelwertspeichers 7, als daß von den digitalen Eingangswerten der Minimalwert eines aufzuwertenden systolischen Zeitintervalls subtrahiert und gegebenenfalls zusätzlich die Adresse des Speicherplatzes (innerhalb eines größeren Speicherbereiches) addiert wird. Der in Figur 1 dargestellte Speicher 9 ist matrixartig organisiert, wobei durch die systolischen Zeitintervalle die Spalten adressiert werden, während durch einen vom Betriebsartenspeicher 2 beeinflussten weiteren Adressendecoder 10 die Zeilen des matrixartig organisierten Speichers adressiert werden. Auf diese Weise wird erreicht, dab jeweils die systolischen Zeitintervalle nur für gleichartige Betriebszustände (Spontanaktionen, ventrikuläre oder AV-sequentielle Stimulation) festgehalten werden. Beim Übergang von einen in den anderen Betriebszustand wird der Mittelwertspeicher 7 über seinen "Reset"-Eingang gelöscht und mit der Mittlung neu begonnen.

Der Speicher 9 wird bei einer Ausführung des Speichers mit kommerziell erhältlichen RAM-Elementen so ausgeführt sein, daß die Decoder 8 und 10 verschiedene Adressleitungen ansteuern, wobei beide Adressensignale eine gemeinsame binäre Adresse bilden, so daß die Adressensteuerung mit derjenigen üblicher Mikroprozessorsysteme übereinstimmt. Die bei den adressierten Speicherwerten vorhandene Grundrate wird mit einem Grundratenspeicher 11 übertragen, in den sie als Pufferspeicher übernommen werden, wobei dieser im Grundratenspeicher enthaltene Wert die minimale Stimulationsfrequenz für die Schrittmacherschaltung festlegt.

Auf diese Weise wird in Abhängigkeit von den ermittelten systolischen Zeitintervallen und der augenblicklichen Betriebsart des Schrittmachers eine minimale Stimulationsrate vorgegeben, welche den ermittelten systolischen Intervallen physiologisch entspricht. Durch die vorgenommene Tabellierung einer — wie nachfolgend zu beschreiben ist — vorher patientenindividuell ermittelten Abhängigkeit ist die Steuerung unabhängig von weiteren die systolischen Zeitintervalle beeinflussenden physiologischen Abhängigkeiten, sofern diese mit der Grundrate zusammenhängen. Wenn sich nämlich die ermittelten Intervall ändern, so wird nach und nach ein Veränderung der Grundrate erfolgen, wobei das zur Einleitung der Veränderung aufgenommene Intervall nicht derjenigen Intervallzeit zu entsprechen braucht, welche zu der einzustellenden Zielfrequenz gehört. Sofern die bis zum Erreichen der Zielfrequenz zu berücksichtigende Änderung der Intervalle nicht das Vorzeichen wechselt. Hiermit wird die dargestellte Steuerung bei festgestellten Abweichungen die Stimulationsfrequenz (bedingt auch durch die Arbeitsweise des Mittelwertspeichers 7) langsam zu der neuen physiologisch bedingten Stimulationsfrequenz hinführen, wobei die im Verlaufe der Annäherung zu laufenden weiteren Intervalländerungen das Erreichen der endgültigen Zielfrequenz nicht stören, wenn der Zusammenhang zwischen aufgenommenem Zeitintervall und zugehöriger Schrittmacherrate eindeutig ist. Der Pufferspeicher für die Grundrate wird jeweils durch ein Signal "Auswertung" aktiviert, welches vom Mittelwertspeicher 7 abgeleitet wird und den Eingang eines neuen systolischen Intervallwertes signalisiert. Bei diesem Betrieb wird davon ausgegangen, daß sämtliche Frequenzwerte im Speicher 9 durch die Kommunikationseinheit 3 von außen übertragen wurden, wobei die Kommunikationseinheit den Schrittmacher über ein Steuerteil 13, welches der externen Programmierung unterliegt, in den Zustand "Auslesen" setzt, so daß jeweils bei der Adressierung durch die systolischen Zeitintervalle der im Speicher 9 vorhandene zugehörige Wert gelesen wird.

Bei einer Betriebsart, die durch das Steuerteil 13 über den Ausgang "Aut" gekennzeichnet wird, erfolgt bei Vorhandensein von Eigenaktionen (entsprechender Ausgang des Schrittmacherteils 1) ein Betrieb mit selbsttätiger Speicherung, der zu bestimmten systolischen Intervallen gehörigen Spontanfrequenz in den Speicher 9, so daß diese Signale bei später wieder notwendig werdender Stimulation mit herangezogen werden können. Der Abstand zwischen zwei aufgenommenen gleichchartigen Spontanktionenn im Herzen (innerhalb eines physiologisch vertretbaren Bereichs) wird in diesem Fall vom Schrittmacherteil 1 in den Grundratenspeicher 11 übertragen und in den jeweils adressierten Speicherplatz eingelesen, der in diesem Fall über den Betriebsartenadressierer 2 Eigenaktionen zugeordnet ist. Dabei wird der Eingang "Speichern" des Speichers 9 über den Ausgang "Aut" des Steuerteils 13 und das Ausgangssignal "Eigenaktionen" 1 des Schrittmachers mittels eines UND-Gatters 14 verknüpft und der Eingang "Speichern" des Speichers 9 über ein ODER-Gatter 15 augesteuert.

Vom Schrittmacherteil 1 wird weiterhin aufgrund der dort aufgenommenen Signale aus dem Herzen eine Tachycardie- (bzw. Extrasystolen-) und Störungserkennung durchgeführt, die mittels einer entsprechenden Baugruppe 15 verarbeitet wird. Bei Auftreten von entsprechenden Zuständen erfolgt eine Sperrung der Verarbeitung der Ausgangssignale des Mittelwertspeichers 7 über den invertierenden Eingang eines in den entsprechenden Ausgang des Mittelwertspeichers 7 eingefügten UND-Gatters 16. Gleichzeitig wird über ein zusätzliches ODER-Gatter in der Rücksetz-Leitung des Mittelwertspeichers 7 dessen Zurücksetzen in den Ausgangszustand veranlaßt.

Bei der zuvor dargestellten Einspeicherung der aktuellen systolischen Invervalle bei Spontanaktionen erfolgt die Steuerung aufgrund der abgespeicherten Werte bei der Stimulation unter der Kontrolle eines zusätzlich vorgesehenen Rechners 12, der ausgelöst durch ein neues im Herzen aufgenommenes Intervallsignal, die zugehörige Grundrate im Speicher 11 aufgrund zusätzlicher Informationen und über die Kommunikationseinheit 3 eingegebener Vorgaben zusätzlich verändern kann, wobei Zugriff auf den Speicher 9 besteht. Diese Veränderung besteht insbesondere in der Anbringung eines Korrekturwertes, welcher bei der Steuerung der Grundrate der Schrittmachers unter Stimulationsbedingungen und Auswertung der entsprechenden Intervalle bei Spontanaktionen, die sich aufgrund der Änderung dieser Bedingungen für das Herz ergebenden Änderung der systolischen Intervalle berücksichtigt. Weiterhin kann eine Interpolation oder ein Ausgleich bei sich ergebenden Frequenzsprüngen bei benachbarten Speicherplätzen ausgeglichen werden. Außerdem lassen sich über die Kommunikationseinheit 3 programmierte Grenzwerte 18 für die Stimulationsfrequenzen vorgeben, welche bei der Nachführung der Grundrate des Schrittmachers nicht überschritten werden. Der entsprechende Grenzwertspeicher wird vom Rechner gelesen und bei Überschreitung des entsprechendes Wertes durch die Steuerung aufgrund der systolischen Invervalle eine Korrektur des im Grundratenspeicher 11 übernommenen Frequenzwertes bewirkt.

In Figur 1a ist der aus den Bausteinen 4 bis 7 bestehende Eingangsteil der Schaltung im Detail wiedergegeben. Dabei besteht die im Mikrofon 4 nachgeschaltete Verarbeitungsgruppe aus einem Eingangsverstärker 51 zur Heraufsetzung der Eingangssignale, einem Filter 52 zur Ausfilterung der für die systolischen Zeitintervalle charakteristischen Frequenzbereiche, einem Schmitt-Trigger 53, einem Gleichrichter und Siebglied 54, der Umwandlung der Frequenzsignale in Impulse einer Polarität, einem Schmitt-Trigger als Schwellwertdiskriminator und einem Impulsformer 55 zur Umwandlung der Vorderflanke des Ausgangsimpulses der Schaltung 54 in einen kurzen Steuerimpuls.

Der Zeitauswertungsteil 6 besteht aus einem monostabilen Multivibrator 61, dem das Ausgangssignal aus der Stufe 5 mittels eines Verzögerungsgliedes 62 um einige Millisekunden verzögert zugeleitet wird. Die Impulsdauer des monostabilen Multivibrators entspricht der maximal auszuwertenden systolischen Intervalldauer. Bei nicht gesetztem Monoflop 61 gelangt das Eingangssignal über ein UND-Gatter 63, dessen weiterer (invertierender) Eingang mit dem Ausgang des Monoflops 61 verbunden ist, zu dem "Setz"-Eingang eines Flip-Flops 64, welches über seinen Q-Ausgang ein UND-Gatter 65 freigibt, so daß ein vom einem Taktsignalgeber 66 erzeugtes Impulssignal zu dem entsprechenden Eingang eines Zählers 67 gelangt, so daß der Zähler entsprechend der abgelaufenen Zeit aufwärts zählt.

Der das systolische Intervall beendende Impuls gelangt bei gesetztem Monoflop 61 über ein UND-Gatter 68 und ein ODER-Gatter 69 zum Rücksetzeingang des Flip-Flops 64, so daß der Zählvorgang unterbrochen wird. Einige Millisekunden verzögert wird vom Ausgang des UND-Gatters 68 her über ein Verzögerungsglied 70 der Eingang "Auslesen" des Zählers 67 aktiviert, so daß der ermittelte Zählerstand in den nachfolgenden Mittelspeicher 7 überführt wird. Nach Ablauf der Impulsdauer des Monoflops 61 wird nach Umsetzung der Rückflanke in einen Steuerimpuls in einem Impulswandler 72 über ODER-Gatter 73 und 69 das Flip-Flop 64 und der Zähler 67 zurückgesetzt, ohne daß ein Auslesen erfolgt. Mittels eines weiteren Monoflops 74, das in die "Setz"-Leitung des Flip-Flops 64 eingefügt ist, wird eine Auswertung der systolischen Zeitintervalle in schnellerer Folge als es der normalen Herzrate entspricht, verhindert, da ein UND-Gatter 75 im Eingang des Monoflops über seinen invertierenden Eingang gesperrt ist, solange dessen Impuls nicht abgeklungen ist. Die Impulsdauer des Monoflops 74 entspricht dabei dem maximalen zu verarbeitenden RR-Abstand der entsprechenden Herzfrequenz.

Der Mittelwertspeicher 7 enthält einen Arithmetikteil 71, der die bisher erfaßten systolischen Intervalle (hier: LVET) mit schwerpunktmäßiger Berücksichtigung der letzten fünf Ereignisse ausmittelt und diesen Wert festhält. Bei Änderung des Betriebszustands bzw. einem Übergang vom stimulierenden Betrieb zu Ruhezustand des Schrittmachers bei vorhandenem Sinusrhythmus wird des Arithmetikteil 71 des Mittelwertspeichers gelöscht, do daß mit den nachfolgend aufgenommenen systolischen Intervallen erneut mit der Mittelwertbildung begonnen wird. Ein Auslesen aus dem Mittelwertspeicher erfolgt mit der Erfassung jeweils eines neuen Mittelwertes, nachdem dieser verarbeitet wurde. (Ausgang: Ansteuerung Rechner und Aktivierung der Ausgabe des Arithmetikteil). Um die Häufigkeit der Veränderung der Stimulationsfrequenz zu verringern, kann hier aber auch eine Herabsetzung der Schaltfrequenz durch Herunterteilen erfolgen. Ein Zähler 76 sperrt mittels eines nachgeschalteten UND-Gatters 77 die Ausgabe von ermittelten Intervallwerten nach einem Wechsel des Betriebszustands, da der Zähler in diesem Fall zurückgesetzt wird (Eingang "Reset") und er ein das UND-Gatter 77 durchschaltendes Ausgangssignal erst dann abgibt, wenn der Zählerstand ≥5 ist.

In Figur 1a ist der aus den Bausteinen 4 bis 7 bestehende Eingangsteil der Schaltung im Detail wiedergegeben. Dabei besteht die im Mikrofon 4 nachgeschaltete Verarbeitungsgruppe aus einem Eingangsverstärker 51 zur Heraufsetzung der Eingangssignale, einem Filter 52 zur Ausfilterung der für die systolischen Zeitintervalle charakteristischen Frequenzbereich, einem Schmitt-Trigger 53, einem Gleichrichter und Siebglied 53, der Umwandlung der Frequenzsignale in Impulse einer Polarität, einem Schmitt-Trigger als Schwellwertdiskriminator und einem Impulsformer 55 zur Umwandlung der Vorderflanke des Ausgangsimpulses der Schaltung 54 in einen kurzen Steuerimpuls.

In Figur 2 ist in der Draufsicht ein Steuerteil 200 für die externe Programmierung und Kontrolle für die Hand des Arztes wiedergegeben. Auf dem Display 201 in der linken Bildhälfte wird die funktionale Abhängigkeit der Herzfrequenz in Abhängigkeit von den zu erfassenden systolischen Intervallen dargestellt, wobei die Wiedergabe jeweils für verschiedene Betriebszustände getrennt (oder überlagert) vorgenommen wird. Die Darstellung entspricht den im Speicher 9 des Schrittmachers festgehaltenen Werten, wobei entweder ein fester funktionaler Zusammenhang vorgegeben werden kann, oder aber die für die im implantierten System ermittelten systolischen Intervalle einzuhaltenden Stimulationsfrequenzen aufgrund der bei vorhandenem Sinusrhythmus aufgefundenen Werten errechnet werden. Auch lassen sich bei vorbestimmten Bereichen der Werte der systolischen Intervalle bevorzugt einzuhaltende Stimulationsfrequenzen einprogrammieren sowie Differenzen der Frequenzen welche zu Intervallwerten bei unterschiedlichen Betriebsarten gehören, um diese Korrekturwerte beim Wechsel des Modes, etc. heranziehen zu können. Die Verarbeitung der Herz- und Stimulationsfrequenzen erfolgt in Form des (reziproken) RR-Invervalls, um hinsichtlich der Verarbeitung Proportionalität zu den erfaßten Intervallzeiten zu erhalten und inverse Zusammenhänge zui vermeiden.

Der in Figur 3 dargestellte Schallempfänger läßt sich sowohl im Schrittmacher selbst als auch in der im Herzen verankerten Elektrode unterbringen. Er enthält einen einseitig eingespannten Piezowandler 301, der über ein mechanisches Koppelelement 302 mit einer eine das Gehäuse bzw. die Wandung 303 abschließenden Membran 304 in Verbindung steht.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen. Insbeondere beschränkt sich die Ausführung nicht auf die Realisierung mit diskreten logischen Baugruppen, sondern läßt sich vorteilhaft auch mit programmierter Logik — vorzugsweise unter Verwendung eines Mikroprozessors — realisieren.

**Patentansprüche**

1. Herzschrittmacher mit Mitteln zur Steuerung der Zeitpunkte der Stimulationsimpulse beim Fehlen von Spontanaktionen mittels mindestens eines innerhalb des Körpers aufgenommenen physiologischen Parameters, wobei die Stimulationsrate jeweils den Zeitpunkt eines auf eine vorangegangene Herzaktion folgenden Stimulationsimpulses festlegt, dadurch gekennzeichnet, daß systolische Zeitintervalle die Bezugsgröße für die Stimulationsrate bilden.

2. Herzschrittmacher nach Anspruch 1, dadurch gekennzeichnet, daß die systolischen Intervalle mittels eines Schallaufnehmers aus dem Herzen abgeleitet werden.

3. Herzschrittmacher nach einem der vorangehenden Ansprüche, dadurch gekennzeichnet, daß als systolisches Zeitintervall mindestens eine der Größen Left ventricular ejection time (LVET), Preejection period (PEP) oder deren Quotient ausgewertet wird.

4. Herzschrittmacher nach einem der vorangehenden Ansprüche, dadurch gekennzeichnet, daß ein, insbesondere piezokeramsiches, Körperschallmikrofon zur Signalaufnahme mit dem implantierbaren Schrittmacher mechanisch verbunden ist.

5. Herzschrittmacher nach Anspruch 4, dadurch gekennzeichnet, daß dem Mikrofon Filter und Zeitselektionsglieder und/oder Mittelwertspeicher nachgeschaltet sind.

6. Herzschrittmacher nach einem der Ansprüche 4 oder 5, dadurch gekennzeichnet, daß das Mikrofon im Schrittmachergehäuse bzw. innerhalb einer mit dem Schrittmacher verbundenen im Herzen zu verankernden Elektrode vorgesehen ist.

7. Herzschrittmacher nach einem der vorangehenden Ansprüche, dadurch gekennzeichnet, daß ein, insbesondere digitaler, Signalspeicher zum Festhalten von Signalen als Zusammenhang der aufgenommenen Intervallwerte und der Herzrate vorgesehen ist.

8. Herzschrittmacher nach Anspruch 7, dadurch gekennzeichnet, daß der Inhalt des Speichers auch durch externe Programmittel veränderbar ist.

9. Herzschrittmacher nach Anspruch 8, dadurch gekennzeichnet, daß in dem Speicher für verschiedene Betriebszustände (Modes) des Schrittmachers getrennt verschiedene funktionale Zusammenhänge festgehalten sind.

10. Herzschrittmacher nach einem der Ansprüche 8 oder 9, dadurch gekennzeichnet, daß der Datenspeicher die Werte in tabelierter Form enthält, wobei eine der Veränderlichen mindestens mittelbar die Adresse für dienenigen Speicherplätze bildet, welche die zugehörigen Datenwerte der anderen Veränderlichen enthalten.

11. Herzschrittmacher nach einem der vorangehenden Ansprüche, dadurch gekennzeichnet, daß vorbestimmte Bereiche der erreichbaren Stimulationsfrequenzen durch externe Programmierung eingrenz- bzw. auswählbar sind.

12. Herzschrittmacher nach Anspruch 11, dadurch gekennzeichnet, daß die Eingrenzung bzw. Auswahl in der Weise erfolgt, daß die Werte derjenigen Stimulationsfrequenzen, die nicht erreicht werden sollen, bei den zu den zugeordneten Speicheradressen, durch die statt dessen einzunehmenden Frequenzwerte ersetzt werden.

13. Herzschrittmacher nach einem der vorangehenden Ansprüche, dadurch gekennzeichnet, daß die Steuerung aufgrund der systolischen Intervalle bei der Erkennung von Extrasystolen, bei Tachycardie und/oder externen Störungen unterbrochen wird.

**Revendications**

1. Simulateur cardiaque doté de dispositifs de commande des instants des impulsions de stimulation en l'absence de réactions spontanées sur le base d'au moins un paramètre physiologique capté à l'intérieur du corps, la fréquence de stimulation déterminant l'instant d'une impulsion de stimulation suivant une réaction cardiaque antérieure, caractérisé en ce que les intervalles systoliques constituent la grandeur de rérerence pour la fréquence de stimulation.

2. Stimulateur cardiaque selon la revendication 1, caractérisé en ce que les intervalles systoliques sont captés au niveau du coeur à l'aide d'un capteur acoustique.

3. Stimulateur cardiaque selon l'une des revendications précédentes, caractérisé en ce que l'on exploite comme intervalle systolique au moins le temps d'éjection ventriculaire gauche (LVET), la période pré-éjection (PEP) ou leur quotient.

4. Stimulateur cardiaque selon l'une des revendications précédentes, caractérisé en ce qu'un microphone pour bruits solidiens, notamment piézocéramique, de captage du signal, est relié mécaniquement au stimulateur cardiaque implanté.

5. Stimulateur cardiaque selon la revendication 4, caractérisé en ce que le microphone est suivi en aval de filtres et d'éléments de sélection temporelle et/ou de mémoires de valeurs moyennes.

6. Stimulateur cardiaque selon l'une des revendications 4 ou 5, caractérisé en ce que le microphone est installé dans le boîtier du stimulateur ou à l'intérieur d'une électrode à implanter dans le coeur et reliée au stimulateur.

7. Stimulateur cardiaque selon l'une des revendications précédentes, caractérisé en ce qu'une mémoire de signal, notamment numérique, est utilisée pour conserver les signaux reliant les intervalles mesurés et la fréquence cardiaque.

8. Stimulateur cardiaque selon la revendication 7, caractérisé en ce que le contenu de la mémoire peut également être modifié par des dispositifs de programmation extérieurs.

9. Stimulateur cardiaque selon la revendication 8, caractérisé en ce que la mémoire stocke des séquences fonctionnelles distinctes pour différents états (modes) de fonctionnement du stimulateur.

10. Stimulateur cardiaque selon l'une des revendications 8 ou 9, caractérisé en ce que la mémoire de données contient les valeurs sous forme de tableau, où l'une des variables constitue au moins indirectement l'adresse des positions de mémoire qui contiennent les valeurs des autres variables.

11. Stimulateur cardiaque selon l'une des revendications précédentes, caractérisé en ce que certaines zones prédéterminées des fréquences de stimulation réalisables peuvent être sélectionnées et délimitées par une programmation extérieure.

12. Stimulateur cardiaque selon la revendication 11, caractérisé en ce que la limitation ou la sélection se fait en remplaçant les valeurs des fréquences de stimulation qui ne doivent pas être atteintes, aux adresses de mémoire qui leurs sont affectées, par les valeurs de fréquence qui doivent être prises à leur place.

13. Stimulateur cardiaque selon l'une des revendications précédentes, caractérisé en ce que la commande opérée sur les intervalles systoliques est interrompue à la détection d'extrasystoles, de tachycardie et/ou de troubles extérieurs.

**Claims**

1. Heart pacemaker with means for controlling the timing of the stimulation pulses in the event of a lack of spontaneous action by means of at least one physiological parameter detected from within the body wherein the stimulation rate determines each time the timing of a stimulation pulse following a preceding heart action, characterised in that the systolic time intervals form the reference quantities for the stimulation rate.

2. Heart pacemaker in accordance with Claim 1, characterised in that the systolic intervals are derived from the heart by means of a sound sensor.

3. Heart pacemaker in accordance with one of the preceding Claims, characterised in that at least one of the quantities left ventricular ejection time (LVET), pre-ejection period (PEP) or their quotient is used as the systolic time interval.

4. Heart pacemaker in accordance with one of the preceding Claims, characterised in that a body-sound microphone and especially a piezoceramic body-sound microphone for receiving the signal is mechanically connected to the implantable pacemaker.

5. Heart pacemaker in accordance with Claim 4, characterised in that filter units and time selection units and/or a mean value memory are connected to the microphone.

6. Heart pacemaker in accordance with one of the Claims 4 or 5, characterised in that the microphone is provided in the pacemaker housing or is provided within an electrode which is to be attached to the heart and which is connected to the pacemaker.

7. Heart pacemaker in accordance with one of the preceding Claims, characterised in that a signal memory and especially a digital signal memory for recording signals is provided to correlate the interval values obtained and the heart rate.

8. Heart pacemaker in accordance with Claim 7, characterised in that the content of the memory can also be changed by external programming means.

9. Heart pacemaker in accordance with Claim 8, characterised in that separate differing functional correlations are held in the memory for the differing operational states (modes) of the pacemaker.

10. Heart pacemaker in accordance with one of the Claims 8 or 9, characterised in that the data memory contains the values in tabulated form wherein one of the variables at least indirectly forms the address for those storage locations which contain the associated data values for the other variables.

11. Heart pacemaker in accordance with one of the preceding Claims, characterised in that the predetermined range of available stimulation frequencies can be limited or selected espectively be external programming.

12. Heart pacemaker in accordance with Claim 11, characterised in that the limiting or selection respectively proceeds in such a fashion that the values of those stimulation frequencies which are not to be available are replaced at the associated memory addresses by the frequency values to be adopted.

13. Heart pacemaker in accordance with one of the preceding Claims, characterised in that control based on the systolic intervals is interrupted by the identification of extra systoles, in tachycardia and/or by external interference.

Fig.1

Fig. 1a

| 1 | 2 | 3 |
|---|---|---|
| 4 | 5 | 6 |
| 7 | 8 | 9 |
|   | 0 | EN-TER |

| Phys. | Eing. | Ausg. | Ein/Aus |
|-------|-------|-------|---------|

201

200

Fig. 2

4

302

304

301

303

Fig. 3